# EUROPEAN PATENT APPLICATION

(11) **EP 1 645 198 A1**
(43) Date of publication of application: **12.04.2006**
(21) Application number: 04732001.5
(22) Date of filing: 10.05.2004
(51) Int. Cl.: A23L 1/28, C12N 1/16, A23L 2/38, C02F 3/34

(54) **PROCESS FOR PRODUCING LIQUID FORMULATION CONTAINING RAW YEAST AND LIQUID FORMULATION**

(30) Priority: 10.07.2003 JP 2003195333
(71) Applicant: Minaki Advance Co., Ltd., Hiroshima-shi, Hiroshima 731-5124 (JP)
(72) Inventor: TANAKA, Yorio, c/o MINAKI ADVANCE CO., LTD., Hiroshima-shi, Hiroshima 7315124 (JP)
(74) Representative: Fuhlendorf, Jörn
(86) International application number: PCT/JP2004/006243
(87) International publication number: WO 2005/004639

(57) **Abstract**

Ingestion of nucleic acid in live state has been enabled by processing live yeast after its freezing to thereby make most of the effects of the nucleic acid and maintain the freshness of the cells. A live yeast is frozen to form a mass of the live yeast, and the mass of the live yeast is cut into particles having a predetermined particle size, and the particles are thawed. The thawed live yeast is then mixed with a diluted aqueous solution of a pineapple enzyme. The aqueous solution of the pineapple enzyme is produced by cutting pineapple into pieces, suspending the pineapple pieces in water to produce a pineapple juice, adding citric acid and yeast to the pineapple juice, heating the mixture, collecting supernatant of the pineapple juice, and diluting the supernatant with water.

## Description

### TECHNICAL FIELD

This invention relates to a health food to be taken by drinking or a water cleaner to be introduced in river or other public water zone, and in particular, to a method for producing a solution containing a live yeast as its component produced by mixing the live yeast and an aqueous solution of a pineapple enzyme, and the solution produced by such method.

### BACKGROUND ART

Live yeast has been widely used as a fermentation material, for example, in producing breads. Live yeast, however, is also known to be helpful in maintaining the health and recovery from diseases since the live yeast contains nucleic acids and they acts as a nutrient. More specifically, nucleic acids are produced in liver, and the function of the liver is said to be weakened with age. Since the nutrient function of the nucleic acids can be supplemented by the live yeast, and by doing so, promotes the curing of the disease and improvement of the immunity, it is now receiving an attention.

The live yeast, however, is highly fermentative, and when it is ingested as it is, fermentation will take place in the stomach and satiety will be felt all the time. Therefore, there has been the problem that the nutrient effects of the nucleic acid in the yeast could not be utilized in an efficient manner.

Among the nucleic acids, ribonucleic acid is known to have enzymatic activity, and the ribonucleic acid combined with aqueous solution of a pineapple enzyme is known to enhance co-enzymatic function. A nutrient solution comprising the aqueous solution of a pineapple enzyme and the live yeast at varying ratio has anti-bacterial properties required in treating allergic traits and pollinosis. Accordingly, there has been a demand for proposal of a solution that enables use of the antibacterial properties of the combination of the pineapple enzyme and the yeast in the form of health food, water cleaner, and the like.

The present invention has been completed for the purpose of overcoming such problems. More specifically, an object of the present invention is to provide a method for producing a solution containing a live yeast as its component and the solution produced by such method which enable ingestion of the nucleic acid in live state by processing the live yeast after freezing to thereby make most of the effects of the nucleic acid, and maintain the freshness of the cells.

### DISCLOSURE OF THE INVENTION

With regard to the production method of the present invention, there is provided a method for producing a solution containing a live yeast as its component, and this method comprises the steps of freezing a live yeast to form a mass of the live yeast, cutting the mass into particles of certain particle size to thaw the particles, and mixing the thawed live yeast with a diluted aqueous solution of a pineapple enzyme.

The mass of the live yeast is preferably frozen at a low temperature of not higher than -25°C, and the live yeast is preferably thawed at a low temperature in the range of 0°C to +5°C. The mass of the live yeast is then cut by a slicer into particles having a particle size of about 0 mm to about 3 mm.

The aqueous solution of a pineapple enzyme is produced bycuttingpineappleintopieces, suspending the pine apple pieces in water to produce a pineapple juice, adding citric acid and yeast to the pineapple juice, heating the mixture, collecting supernatant of the pineapple juice, and diluting the supernatant with water.

Alternatively, the aqueous solution of a pineapple enzyme is preferably produced by cutting pineapple into pieces, suspending the pineapple pieces in degassed water or deep ocean water to produce a pineapple juice, adding citric acid and yeast to the pineapple juice, heating the mixture at 60°C, collecting supernatant of the pineapple juice, and diluting the supernatant with water.

In the production method as described above, the live yeast is kept alive without being killed when the mass of the live yeast is cut in frozen state, and pieces with minimized size can be produced with the live yeast being alive. Cutting of the yeast mass in frozen state also prevents fermentation of the live yeast, and the drug produced by the method of the present invention can be ingested without feeling satiety. In addition, the mass of the live yeast solidified by freezing is a ice-like mass, and therefore, the mass is easily cut by a slicer to produce particles having the predetermined particle size.

With regard to the solution of the present invention, a solution containing live yeast as its component is provided, and this solution comprises: a live yeast produced by freezing a live yeast to form a mass of the live yeast, cutting the mass into particles of certain particle size, and thawing the particles; and an aqueous solution of a pineapple enzyme produced bycuttingpineappleintopieces, suspending the pineapple pieces in water to produce a pineapple juice, adding citric acid and yeast to the pineapple juice, heating the mixture, collecting supernatant of the pineapple juice, and diluting the supernatant with water. The content of the live yeast in the solution is higher than that of the aqueous solution of the pineapple enzyme.

Alternatively, the content of the live yeast in the solution may be lower than that of the aqueous solution of the pineapple enzyme.

The mixture of the live yeast and the aqueous solution of the pineapple enzyme preferably further comprises a sweetener such as sugar or honey added thereto.

In the solution as described above, the ribonucleic acid among the nucleic acids combined with the aqueous solution of a pineapple enzyme having an enzymatic function is capable of enhancing co-enzymatic functions. The nutrient comprising the aqueous solution of a pineapple enzyme and the live yeast in various ratio has anti-bacterial properties required in treating allergic trait and pollinosis.

Since the aqueous solution of a pineapple enzyme has added thereto citric acid solution and yeast added which promote the catalytic functionof activating themicroogganism, the solution is less likely to undergo inactivation in the contaminated water. Accordingly, when this solution is dispersed in a contaminated water, colonies of organisms which enable survival of such organisms in the contaminated water will be formed after a predetermined time to facilitate survival of the microorganisms. These microorganisms themselves take and release the enzyme into and out of their body and to facilitate decomposition of substances such as organic compounds, nitrogen oxides, and sulfides so that the oxidized water is reduced thereby improving the water quality.

### BRIEF DESCRIPTION OF THE DRAWINGS

FIG. 1 is a block diagram showing the method for producing a solution containing a live yeast as its component according to the present invention.
FIG. 2 is a table showing the composition of the live yeast.
FIG. 3 is a table showing the composition of the aqueous solution of the pineapple enzyme.
FIG. 4 is a table showing propagation of the live yeast in a contaminated water (sludge).

### BEST MODE FOR CARRYING OUT THE INVENTION

Next, preferred embodiments of the present invention are described by referring to the drawings.

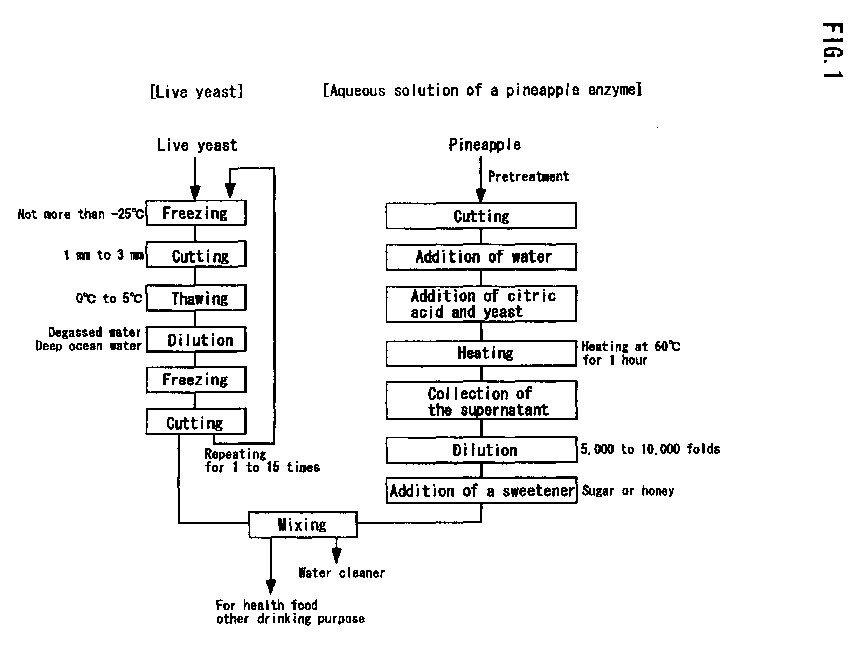

FIG. 1 is a block diagram showing the method for producing a solution containing a live yeast as its component according to the present invention.

The method for producing a solution containing a live yeast as its component of the present invention comprises the steps of freezing a live yeast such as live baking yeast to form a mass of the live yeast, cutting this mass of live yeast into particles having a predetermined particle size, and thawing the particles; and mixing the thawed live yeast with an aqueous solution of a pineapple enzyme. The live yeast is used in the present invention since it contains a large amount of nucleic acid.

The live yeast is usually stored at about -1°C to about -5°C. A 500 g mass of this live yeast is solidified by freezing this mass at a low temperature of not higher than -25°C since the live yeast can be processed alive without killing the yeast when the live yeast mass is cut in frozen state. Freezing of the live yeast also prevents fermentation of the live yeast, and a health food produced from such live yeast can be ingested without feeling satiety.

The solidified frozen mass of the live yeast is then cut into particles each having a particle size of about 1 mm to 3 mm by a slicer. This operation of cutting the frozen five yeast particles with the slicer is repeated several times to reduce the size of each of the particles. Since the live yeast mass is in the state of an ice mass in the production method of the present invention, the live yeast mass is readily sliced.

Next, the live yeast particles prepared by cutting is diluted in 2 liters of degassed water or deep ocean water, and the dilution is solidified by freezing. Such degassed water or deep ocean water is used in the dilution since use of the degassed water or the deep ocean water is advantageous in increasing the clarity of the dilution compared to the use of tap water containing chlorine. The degassed water or the deep ocean water is used at an amount of 2 liters per 500 g of the live yeast.

The frozen live yeast is further cut with the slicer, and this operation of cutting with the slicer of the live yeast, thawing, and freezing is repeated once at the least and about 15 times at the most.

In the meanwhile, the aqueous solution of a pineapple enzyme is produced by using an unripe green pineapple for the staring material. A green pineapple is used since the pineapple enzyme is often denatured in the ripe, more yellowish pineapple, and amount of the enzyme extracted would not be as much in such ripe pineapple. The pineapple is cut into the small pieces with the size as small as 3 to 5 cm³. The core which is the fiber-rich part is used.

The pineapple pieces are placed in a degassed water or in deep ocean water to produce the pineapple juice. Such degassed water or deep ocean water is used since use of the degassed water or the deep ocean water is advantageous in increasing the clarity of the juice compared to the use of tap water containing chlorine. The degassed water or the deep ocean water is used at an amount of 5 liters per 1 pineapple (about 1 kg).

Next, citric acid (about 30 g) and yeast (about 2 g) are added to the pineapple juice. For example 50 cc (a powder of about 1 g) of yeast is added to 5 liters of pineapple juice with stirring. This pineapple juice is heated at 60°C for about 1 hour to produce the aqueous solution of a pineapple enzyme. The yeast mixed with the pineapple juice may be dried yeast or liquid yeast instead of the live yeast.

About 7 liters of stock solution for health food or water cleaner containing the live yeast as its component can be produced from one pineapple. This stock solution is used after diluting it 5,000 to 10,000 times.

To this aqueous solution of a pineapple enzyme is added a sweetener such as sugar or honey at an amount of about 3% by weight in relation to the total weight. Next, to this aqueous solution of a pineapple enzyme is added about 0.5 to about 2 g of the live yeast that has been produced as described above to complete the production of the solution which is used as a health food or a water cleaner.

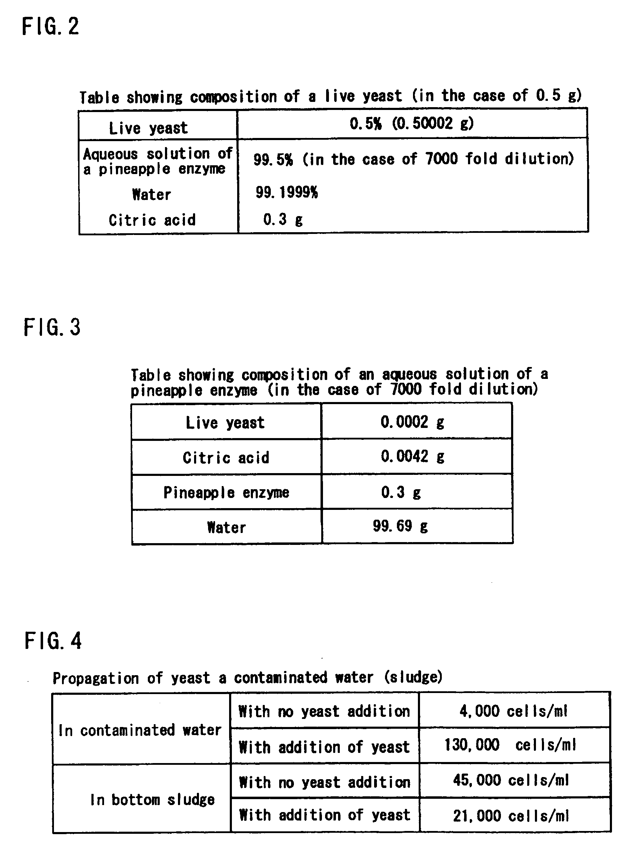

FIG. 2 is a table showing the composition of the live yeast.

Depending on the intended use, namely, whether the solution is to be used as a health food or a water cleaner, for example, the solution may be produced to have the content of the live yeast higher than that of the aqueous solution of the pineapple enzyme, or to have the content of the live yeast lower than that of the aqueous solution of the pineapple enzyme. Mixing ratio of the solution produced by such production method may be varied as shown in the composition table of FIG. 2 so that it contains the "live yeast" at a higher content or as shown in the composition table of FIG. 3 so that it contains the "aqueous solution of the pineapple enzyme" at a higher content.

In the production method of the present invention, nucleic acids can be ingested alive since the live yeast are cut, and the effects of the nucleic acid is drawn at its best. Freezing also enables to main freshness of the cell. When the solution of the present invention is used as a health food, ingestion of 1.5 g to 2 g per day is sufficient for the development of the effect. The solution is most effective for cancer and adult diseases such as diabetes.

Next, results of the trial in which people ingested the solution of the present invention as a health food are described.

First, the cases of drinking the solution having a higher content of the "live yeast" are described.
(a) Patient suffering from pharyngeal cancer (a male of 60 year old)
   A patient suffering from pharyngeal cancer took the "live yeast" solution of the present invention at a daily dose of 0.5 g. Side effects of anticancer agent disappeared from the first day and this patient started taking the meal. After 1 months, daily dose was increased to 1 g/day, and the 90% of the cancer cells disappeared after 2 months.
(b) 4 lung cancer patients (males in their 50' s and 60' s)
   Patients suffering from lung cancer took the "live yeast" solution of the present invention at a daily dose of 2 g. The effects on cancer became apparent in 2 to 3 weeks. The cancer was completely cured after 3 months of drinking.
(c) Liver cancer (a female in her 40's)
   A patient suffering from lung cancer took the "live yeast" solution of the present invention at a daily dose of 0.5 g. The cancer was completely cured after drinking for 1 month.
(d) Kidney cancer (a female in her 40's).
A patient suffering from kidney cancer took the "live yeast" solution of the present invention at a daily dose of 0.5 g. The cancer was completely cured after drinking for 2 months.
(e) Liver cirrhosis (a male of 66 year old)
   A patient suffering from terminal liver cirrhosis and declared with the life expectancy of 1 month took the "live yeast" solution of the present invention at a daily dose of 1.5g. Except for the period of dementia due to generation of ammonia in brain, the patient recovered to such state that he was able to take a meal after 3 days of drinking the solution, and the patient could leave the hospital at the third week after the starting of drinking.
(f) Diabetes (a male of 55 year old, a practitioner)
   When a person with a blood glucose level 480 mg/dl with several year history of the disease took the "live yeast" solution of the present invention at a daily dose of 2 g, the blood glucose level decreased to normal level at three days after the starting of drinking. This man kept drinking the solution for 1 month, and the diabetes has not recurred for 4 years since then.
(g) Diabetes (a male of 56 years old)
   When a person with a blood glucose level 233 mg/dl took 0.5 g of the "live yeast" solution of the present invention once, the blood glucose level decreased to 28 mg/dl.
(h) Allergy (a male of 38 year old)
   When a person suffering from severe allergy with the doctor stop took the "live yeast" solution of the present invention at a daily dose of 2 g, the symptoms improved in 1 month and the rough skin recovered to normal skin.
(i) Maxillary empyema (a female of 35 year old)
   A person with a 5 year history of maxillary empyema and undertreatmentofheadache, faceache, andindispositionbecause the puss accumulates at a hardly accessible position took the "live yeast" solution of the present invention at a daily dose of 1 g, the various symptoms recovered in 1 month.
   Next, the cases of drinking the solution having a higher content of the "aqueous solution of a pineapple enzyme" as a health food are described.
(j) Diabetes patient (a male in his 50's)
   Apersonsuffering fromdiabetes took the "aqueous solution of a pineapple enzyme" solution of the present invention at a daily dose of 360 cc. The diabetes was completely cured after 2 months.
(k) Urticaria allergy (a male of 43 year old)
   A person suffering fromurticaria allergy took the "aqueous solution of a pineapple enzyme" solution of the present invention at a daily dose of 360 cc. The allergy was completely cured after 2 months.
(l) Liver trouble (a male of 25 year old)
   A person suffering from a liver trouble took the "aqueous solution of a pineapple enzyme" solution of the present invention at a monthly dose of 20 liters. The disease was completely cured after 3 months.
(m) Allergy (a male of 28 year old)
   A person suffering from allergy took the "aqueous solution of a pineapple enzyme" solution of the present invention at a monthly dose of 20 liters. The itch disappeared , and drug administration was no longer necessary.
(n) Allergy (a male of 16 months)
   An infant suffered from severe allergy with the body fluid exuding from all over the face, and the doctor instructed that the infant could only take rice and whitefish and the infant could not be weaned. The "aqueous solution of a pineapple enzyme" solution of the present invention was spread on the infant face, and on the day 4, the condition recovered to the state that no body fluid was exuding and no itch was felt.
(o) Allergy (a female of 10 months)
   In an infant suffering from allergy on the face and hand, the "aqueous solution of a pineapple enzyme" solution of the present invention was directly spread on the lesion. The condition cured in 1 weak.
(p) Pollinosis (a large number of males and females in their 10' s to 60's)
   Those suffering from pollinosis was treated with the "aqueous solution of a pineapple enzyme" solution of the present invention. Those secreting nasal discharge were applied with the solution, and also, orally administered with the solution at a daily dose of 180 cc. Those secreting tears was instilled with the solution, and also, orally administered with the solution at a daily dose of 180 cc. In the case of those instilled with the solution, excessive tear secretion stopped on the day of the instillation or after 3 days at the latest, and the symptom improved.

In addition to drinking as a health food, the solution of the present invention may also be used as a cleaner introduced in a contaminated water such as river water to thereby improve the quality of the water. The live yeast and the aqueous solution of a pineapple enzyme incorporated in the solution of the present invention are capable of promoting catalytic functions that activate microorganisms in the contaminated water. Accordingly, when a minute amount of the solution of the present invention is added to the contaminated water, colonies (viable spheres) of the microorganisms living in the contaminated water are formed in approximately 3 months to facilitate survival of the microorganisms in the contaminated water. These microorganisms ingests and releases enzymes into and out of their cell to carry out the decomposition reaction and the water quality is thereby improved by the decomposition of the toxic substances and the reduction of the oxidized water.

The nucleic acid contained in the solution of the present invention is capable of promoting catalytic functions. For example, planktons are required for the cycling and survival of the microorganisms and a complex of silicon is required for the development of the planktons. Iron is necessary for various functions of a microorganism, and a metal ion is capable of suppressing hydrogen sulfide. The solution of the present invention may be added dropwise to the contaminated waters of a river, pond, lake, dam, harbor, coastal area, or other public water zone to thereby improve water quality of the contaminated water. Alternatively, the solution of the present invention may be added to a contaminated water such as sewage or waste water from factories or installation provided in a plant to thereby improve water quality of the contaminated water.

FIG. 4 is a table showing propagation of the yeast in the contaminated water (sludge).

More specifically, the yeast which can survive both in the presence and absence of oxygen is useful in activating other microorganisms living in the area of contaminated water. This phenomenon can replace the role of the photosynthetic bacteria in the area of contaminated water where the photosynthetic bacteria can not survive. As shown in the table of FIG. 4, the propagation of the photosynthetic bacteria was greatly different between the case where the solution of the present invention had been added to the contaminated water and the case where the solution had not been added to the contaminated water, or between the case where the solution of the present invention had been added to the sludge on the bottom of the contaminated water and the case where the solution had not been added to the sludge on the bottom of the contaminated water.

The method for producing a solution containing a live yeast as its component is not limited for the type of the pineapple enzyme used. In other words, the pineapple enzyme is not limited to the one extracted from pineapple juice as long as it comprises a pineapple enzyme and it is mixed with the yeast and citric acid which promotes catalytic function of activating the microorganisms. More specifically, any pineapple enzyme may be used as long as the enzyme is the one which is readily extracted at a low cost and exemplary such enzymes include "papain" found in papaya, "cathepsin" found in animal cells, and "phosphatase" found in a plant cells.

While the drug of the present invention has been described by referring to the embodiment wherein the present drug is ingested as a health food mainly taken by drinking, the present drug may also be directly applied to the skin or instilled in the eye.

In the method for producing the live yeast-containing solution of the present invention, the mass of the live yeast is cut into particles after freezing. As a consequence, the small sized particles comprises live microorganisms, and fermentation of the live microorganisms is prevented. Accordingly, the solution can be ingested without feeling satiety.

The live yeast used in the solution of the present invention is quite inexpensive compared to the expensive nucleic acid, and combination of such live yeast with the pineapple enzyme and further dilution of the mixture has enabled a large-scale, inexpensive production of the solution and full utilization of the advantages effects. The solution is particularly effective for cancer and adult diseases such as diabetes.

The solution of the present invention may also be used as a water cleaner. The solution does not cause contamination when introduced in public water zone such as river, and the solution is useful in protecting water resources.

## Claims

1. A method for producing a solution containing a live yeast as its component, comprising the steps of:
freezing a live yeast to form a mass of the live yeast, cutting the mass of the live yeast into particles having a predetermined particle size, and thawing the particles; and
mixing the thawed live yeast with a diluted aqueous solution of a pineapple enzyme.

2. The method for producing the live yeast-containing solution according to claim 1, wherein the live yeast is frozen at a low temperature of not higher than -25°C.

3. The method for producing the live yeast-containing solution according to claim 1, wherein the live yeast is thawed at a low temperature in the range of 0°C to +5°C.

4. The method for producing the live yeast-containing solution according to claim 1, wherein the frozen mass of the live yeast is cut by a slicer into particles having a particle size of about 0 mm to about 3 mm.

5. The method for producing the live yeast-containing solution according to claim 1, wherein the aqueous solution of a pineapple enzyme is produced by cutting pineapple into pieces, suspending the pineapple pieces in water to produce a pineapple juice, adding citric acid and yeast to the pineapple juice, heating the mixture, collecting supernatant of the pineapple juice, and diluting the supernatant with water.

6. The method for producing the live yeast-containing solution according to claim 1, wherein the aqueous solution of a pineapple enzyme is produced by cutting pineapple into pieces, suspending the pineapple pieces in degassed water or deep ocean water to produce a pineapple juice, adding citric acid and yeast to the pineapple juice, heating the mixture at 60°C, collecting supernatant of the pineapple juice, and diluting the supernatant with water to a predetermined concentration.

7. A solution containing live yeast as its component, comprising:
a live yeast produced by freezing a live yeast to form a mass of the live yeast, cutting the mass into particles of certain particle size, and thawing the particles; and
an aqueous solution of a pineapple enzyme produced by cutting pineapple into pieces, suspending the pineapple pieces in water to produce a pineapple juice, adding citric acid and yeast to the pineapple juice, heating the mixture, collecting supernatant of the pineapple juice, and diluting the supernatant with water,
wherein content of the live yeast in the solution is higher than that of the aqueous solution of the pineapple enzyme.

8. A solution containing live yeast as its component, comprising:
a live yeast produced by freezing a live yeast to form a mass of the live yeast, cutting the mass into particles of certain particle size by repeating the cutting operation for a plurality of times, and thawing the particles; and
an aqueous solution of a pineapple enzyme produced by cutting pineapple into pieces, suspending the pineapple pieces in water to produce a pineapple juice, adding citric acid and yeast to the pineapple juice, heating the mixture, collecting supernatant of the pineapple juice, and diluting the supernatant with water,
wherein content of the live yeast in the solution is lower than that of the aqueous solution of the pineapple enzyme.

9. The live yeast-containing solution according to claim 7 or 8, wherein the mixture of the live yeast and the aqueous solution of the pineapple enzyme further comprises a sweetener such as sugar or honey added thereto.
